# EUROPEAN PATENT SPECIFICATION

(11) **EP 2 335 074 B1**
(45) Date of publication and mention of the grant of the patent: **20.03.2013**
(21) Application number: 08875930.3
(22) Date of filing: 26.09.2008
(51) Int. Cl.: G01N 33/574, G01N 33/94

(54) **INDIVIDUAL 5-FLUOROURACILE DOSE OPTIMIZATION IN FOLFOX TREATMENT**
OPTIMIERUNG DER 5-FLUORURACIL-EINZELDOSIS BEI FOLFOX-BEHANDLUNG
OPTIMISATION DE DOSE DE 5-FLUORO-URACILE INDIVIDUELLE DANS LE TRAITEMENT PAR FOLFOX

(43) Date of publication of application: 22.06.2011
(73) Proprietor: Institut de Cancérologie de l'Ouest (I.C.O), 49933 Angers Cedex 9 (FR); UNIVERSITE D'ANGERS, 49000 Angers (FR)
(72) Inventor: GAMELIN, Erick, F-49080 Bouchemaine (FR); BOISDRON-CELLE, Michèle, F-49170 Saint-Léger-des-Bois (FR); MOREL, Alain, F-49610 Juigné-sur- Loire (FR)
(74) Representative: Regimbeau
(86) International application number: PCT/IB2008/055625
(87) International publication number: WO 2010/035075

(56) References cited:
- GAMELIN ERICK ET AL: "Individual fluorouracil dose adjustment based on pharmacokinetic follow-up compared with conventional dosage: results of a multicenter randomized trial of patients with metastatic colorectal cancer." JOURNAL OF CLINICAL ONCOLOGY : OFFICIAL JOURNAL OF THE AMERICAN SOCIETY OF CLINICAL ONCOLOGY 1 MAY 2008, vol. 26, no. 13, 1 mai 2008 (2008-05-01), pages 2099-2105, XP002528592 ISSN: 1527-7755
- YCHOU MARC ET AL: "Individual 5-FU dose adaptation in metastatic colorectal cancer: results of a phase II study using a bimonthly pharmacokinetically intensified LV5FU2 regimen." CANCER CHEMOTHERAPY AND PHARMACOLOGY OCT 2003, vol. 52, no. 4, octobre 2003 (2003-10), pages 282-290, XP002528593 ISSN: 0344-5704
- GAMELIN E ET AL: "5-FU dose monitoring and prevention of oxaliplatin-induced neurotoxicity in FOLFOX 4 regimen: Results of a phase II study." ASCO GASTROINTESTINAL CANCERS SYMPOSIUM, [Online] janvier 2008 (2008-01), XP002528594 Extrait de l'Internet: URL:http://opd.asco.org/ASCOv2/Meetings/Ab stracts?&vmview=abst_detail_view&confID=53 &abstractID=10320> [extrait le 2009-05]

## Description

The present disclosure belongs to the field of improved personalized medicine. More precisely, the present disclosure relates to a method for progressively optimizing the 5-FU dose administered by continuous infusion in patients treated by a FOLFOX regimen or a similar regimen, based on the 5-FU plasmatic concentration measured during the previous 5-FU continuous infusion and on a herein described decision algorithm. The present disclosure also relates to a method for treating a cancer patient in which the 5-FU dose administered in continuous infusion in each FOLFOX or similar treatment cycle is optimized using the decision algorithm according to the invention.

### BACKGROUND ART

Most drugs may have deleterious effects. However, anticancer drugs are among those resulting in the worse adverse effects. Indeed, anticancer drugs are usually cytotoxic active agents with some preference for tumor cells. However, they also display some toxicity on other cells, thus resulting in often serious adverse reactions (20-25% of grade 3-4 toxicity and 0.2% mortality).

This is an important problem, since serious adverse effects not only affect patients' life quality, but may also result in death due to toxicity, or more often to the end or decrease of the treatment, thus decreasing its efficiency.

Interindividual metabolism variations, which influence drugs anabolism and catabolism capacities, participate to the toxicity risk. However, despite some recent improvements of the knowledge concerning anticancer drugs metabolism and of pharmacological technologies, therapeutic individualization is not yet common practice.

In contrast, doses are usually standardized. Although doses and protocols standardization may have been once useful, it now shows its limits concerning efficiency and toxicity of the treatment, depending on the treated subject.

However, the administered dose of anticancer drug is usually still calculated depending on body surface, which relevancy is based neither on experimental or theoretical justification, and at best on a few biological tests such as complete blood count and renal check-up. Individual pharmacokinetic, metabolic, genetic or epigenetic particularities are not taken into account.

There is thus a need for treatment methods using anticancer compounds in which such individual particularities would be taken into account in order to decrease toxicity and improve efficiency of the treatment.

5-fluorouracile (5-FU) is the leading anticancer drug of fluoropyrimidine family, a therapeutic class of agents interfering with DNA synthesis. 5-FU is a major chemotherapeutic drug, and is notably used in the treatment of colorectal cancer, gastric cancer, oesophagi cancer, ORL cancer, and breast cancer, particularly as an adjuvant treatment or in metastatic situations. Each year, more than 90 000 patients are treated by 5-FU.

However, 5-FU results in 20-25% of severe grade 3-4 toxicity, including toxicities in the digestive tract, such as diarrhea, which may be bloody or hemorrhagic; haematopoietic complications, such as leuco-neutropenias, which may result in superinfection or septicaemia; skin or mucosa complications, such as mucites, hand-foot syndrome; toxidermia; cardiac toxicity and a cerebellum syndrome.

Such adverse effects may be combined with each other, resulting in a polyvisceral toxicity scheme, with is very early in 5-8% of patients and even gives rise to death in 0.8% of treated patients. These adverse effects may also appear later, during the treatment.

5-FU is usually used in metastatic situations. In addition, it is also more and more often used as an adjuvant treatment, i.e. in the case of patients treated for a localized tumor for which a relapse is feared. The risk of a severe toxic adverse effect cannot be taken in such conditions.

The adverse effects of 5-FU are mainly due to a great interindividual variability of 5-FU metabolism. 5-FU cytotoxicity mechanism is based on its conversion in active nucleotides that block DNA synthesis. Such active nucleotides are obtained when 5-FU is metabolised by the anabolic pathway. However, there is an equilibrium between 5-FU enzymatic activation (anabolic pathway) and 5-FU elimination in the catabolic pathway. The initial and limiting enzyme of 5-FU elimination (catabolic pathway is dihydropyrimidine dehydrogenase (DPD). This ubiquitous enzyme is a major factor of 5-FU biodisponibility, since in a subject with normal DPD enzymatic activity, about 80% of administered 5-FU is eliminated by DPD in the catabolic pathway, while only 20% of administered 5-FU is available for the anabolic pathway that us necessary for its cytotoxic action.

However, in patients with a deficiency (total or partial) in DPD activity, the percentage of administered 5-FU that is available for the anabolic pathway that is necessary for its cytotoxic action is greatly increased, and these patients thus have an increased risk of developing acute, early and severe 5-FU toxicity.

On the other hand, in patient with an increased DPD activity a standard dose based on the body surface area is insufficient and consequently inefficient.

DPD activity shows a great interindividual variability, with measured activity values that may differ from a 6 times ratio between two distinct patients (Etienne M C, et al: J Clin Oncol 12: 2248-2253, 1994). This enzymatic variability results in a great variability in 5-FU metabolism and plasmatic kinetics, since 5-FU clearance varies of a factor 6 to 10 depending on the subject (Gamelin E., et al.. J Clin Oncol, 1999, 17, 1105-1110; Gamelin E., et al. J. Clin. Oncol., 1998, 16 (4), 1470-1478).

This situation has enormous implications for treatment toxicity, but also for treatment efficiency. Indeed, several studies have shown that pharmacokinetic parameters are correlated with toxicity but also with treatment efficiency, notably concerning tumor response in colorectal and ORL cancers.

In addition, it has been found that the range of plasmatic 5-FU concentration in which the treatment is efficient and does not lead to severe adverse effects is rather narrow, so that there is not much difference between efficient and toxic plasmatic 5-FU concentrations.

There is thus a need for treatment methods that would take such variability into account in order to administer to each patient a 5-FU dose that will result in a plasmatic 5-FU concentration in the narrow range in which it is both sufficient to have therapeutic activity and is low enough to prevent severe grade 3-4 toxicities.

In addition to DPD activity variability, 5-FU metabolism also highly depends on the administered dose and mostly on administration duration, i.e. on perfusion duration. Indeed, DPD is saturable, so that a patient's plasmatic kinetics is not linear, and clearance is multiplied by a factor 10 when changing from a bolus administration to a continued perfusion during several hours or days (Gamelin E., Boisdron-Celle M. Crit Rev Oncol Hematol, 1999, 30, 71-79).

A general individual optimization method of 5-FU dose cannot thus be provided. In contrast, although some tolerance may apply for small variations, a particular individual optimization method of 5-FU dose has to be found for each 5-FU treatment protocol, depending on the dose and mostly duration of 5-FU administration.

In addition, the increase or decrease in 5-FU plasmatic concentration in a patient is not proportional to the increase or decrease of the dose of 5-FU that is administered to said patient, so that it is not easy to determine how much to increase or decrease the administered 5-FU dose in order to reach a particular 5-FU plasmatic concentration when starting from a higher or lower concentration obtained with a given administered 5-FU dose.

Moreover, although 5-FU was at some time used in monotherapies, it is now usually administered in combination with other cytotoxic agents, such as oxaliplatine or irinotecan, and optionally with additional targeted therapies using monoclonal antibodies, such as cetuximab, panitumumab or bevacizumab.

These additional agents, and particularly chemotherapeutic agents such as oxliplatin or irinotecan, may also generate adverse effects, which may be similar to those induced by 5-FU, thus creating a risk of synergism in toxicity development as well as in tumor treatment.

In particular, oxaliplatin may notably induce diarrhea and leucopenia, which are already conventional toxicities induced by 5-FU (Graham J, Mushin M, Kirkpatrick P. Oxaliplatin. Nat Rev Drug Discov, 2004, 3: 11-12). Furthermore, it has been shown that oxaliplatin inhibits 5-FU metabolism (Maindrault-Goebel F, et al. Oncology Multidisciplinary Research Group (GERCOR).Ann Oncol. 2000 Nov;11 (11):1477-83).

As a result, depending on the chemotherapeutic agent that is used in combination with 5-FU, a particular individual optimization method of 5-FU dose has to be found. Such a method should determine the range in which the 5-FU plasmatic concentration is

Some attempts to optimize the 5-FU dose administered to patients in anticancer protocols have been made. However, as mentioned above, results are not transposable to other protocols, in particular if the administration mode (and notably the duration of the continuous infusion) of 5-FU is changed, or if 5-FU is combined with a chemotherapeutic agent that may influence 5-FU pharmacokinetics such as oxaliplatin.

Gamelin et al (Gamelin, E et al. J Clin Oncol. 2008 May 1;26(13):2099-105) defined a method for adapting 5-FU dose in a treatment based on weekly administration of folinic acid combined with 5-FU in an 8 hours continuous infusion. However, such a protocol is no more used, since current protocols generally combine 5-FU with folinic acid and another chemotherapeutic drug, generally oxaliplatin or irinotecan. In addition, current protocols use much longer continuous infusions of 5-FU.

Ychou et al (Ychou M, Duffour J, Kramar A, et al. Cancer Chemother Pharmacol, 2003, 52: 282-90.) describe a method for increasing 5-FU dose in a treatment based on a bimonthly LV5FU2 regimen: . However, such a protocol is also no more used, since current protocols generally combine 5-FU with folinic acid and another chemotherapeutic drug, generally oxaliplatin or irinotecan. In addition, the method described in Ychou et al only intends to increase the 5-FU dose, and an increase is systematically applied unless a significant (grade II-IV) toxicity is observed. Thus, although this method permits to increase the 5-FU dose and potentially to increase treatment efficiency, it does not permit to prevent severe toxicity by remaining in the narrow window in which 5-FU plasmatic levels are efficient but not toxic. The method of Ychou et al thus still make the patient take a significant risk, which is not acceptable in first line treatment.

In the present application, the inventors have found a method for optimizing the next 5-FU dose to be administered by continuous infusion to a patient treated with a FOLFOX protocol (5-FU in bolus and continuous infusion of 46 hours, folinic acrid, and oxaliplatin), based on the plasmatic 5-FU concentration measured from a blood sample taken before the end of the 5-FU perfusion, and on a new decision algorithm.

### DESCRIPTION OF THE INVENTION

The present invention thus concerns a method for determining from a blood sample of a patient suffering from cancer the dose D(n+1) of 5-fluorouracile (5-FU) for the next cycle of treatment (n+1), wherein
- each treatment cycle i comprises:
   - 0-500 mg/m² of 5-fluorouracile (5-FU) administered in a bolus,
   - 0-600 mg/m² of folinic acid or a salt thereof,
   - a dose D(i) (in mg/m²) of 5-FU administered in a continuous infusion of 43 to 49 hours, and
   - 70-130 mg/m² of oxaliplatin; and
- said blood sample has been taken from said patient in previous treatment cycle n at least 2 hours after the beginning of the 5-FU perfusion and before the end of said perfusion,
said method comprising:
- dosing in vitro the 5-FU plasmatic concentration ([5-FU]) in the blood sample
- calculating D(n+1) depending on D(n) using the following decision scheme:
   - if [5-FU] < 100 µg/L, then D(n+1) = D(n) × 1.40,
   - if 100 ≤ [5-FU] < 200 µg/L, then D(n+1) = D(n) × 1.30,
   - if 200 ≤ [5-FU] < 300 µg/L, then D(n+1) = D(n) × 1.20,
   - if 300 ≤ [5-FU] < 400 µg/L, then D(n+1) = D(n) × 1.10,
   - if 400 ≤ [5-FU] < 550 µg/L, then D(n+1) = D(n) × 1.05,
   - if 550 ≤ [5-FU] ≤ 600 µg/L, then D(n+1) = D(n),
   - if 600 < [5-FU] < 700 µg/L, then D(n+1) = D(n) × 0.95,
   - if 700 ≤ [5-FU] < 800 µg/L, then D(n+1) = D(n) × 0.90,
   - if 800 ≤ [5-FU] < 900 µg/L, then D(n+1) = D(n) × 0.85,
   - if [5-FU] ≥ 900, then D(n+1) = D(n) × 0.80.

The present disclosure also relates to a method for treating a patient suffering from cancer, comprising:
- Administering to said patient successive treatment cycles, in which each treatment cycle i comprises:
   - 0-500 mg/m² of 5-fluorouracile (5-FU) administered in a bolus,
   - 0-600 mg/m² of folinic acid or a salt thereof,
   - a dose D(i) of 5-FU (in mg/m²) administered in a perfusion of about 46 hours, and
   - 70-130 mg/m² of oxaliplatin.
- At each cycle i, taking a blood sample from the patient at least 3 hours after the beginning of the 5-FU perfusion and before the end of said perfusion, and dosing in vitro the 5-FU plasmatic concentration ([5-FU]),
   wherein
- the initial dose D(1) at treatment cycle 1 is at most 2500 mg/m²
- at each cycle i, the next dose D(i+1) of the next treatment cycle i+1 is determined using the following decision scheme:
   - if [5-FU] < 100 µg/L, then D(n+1) = D(n) × 1.40,
   - if 100 ≤ [5-FU] < 200 µg/L, then D(n+1) = D(n) × 1.30,
   - if 200 ≤ [5-FU] < 300 µg/L, then D(n+1) = D(n) × 1.20,
   - if 300 ≤ [5-FU] < 400 µg/L, then D(n+1) = D(n) × 1.10,
   - if 400 ≤ [5-FU] < 550 µg/L, then D(n+1) = D(n) × 1.05,
   - if 550 ≤ [5-FU] ≤ 600 µg/L, then D(n+1) = D(n),
   - if 600 < [5-FU] < 700 µg/L, then D(n+1) = D(n) × 0.95,
   - if 700 ≤ [5-FU] < 800 µg/L, then D(n+1 ) = D(n) × 0.90,
   - if 800 ≤ [5-FU] < 900 µg/L, then D(n+1) = D(n) × 0.85,
   - if [5-FU] > 900, then D(n+1) = D(n) × 0.80.

The methods according to the invention thus concern cancer patients treated by a FOLFOX regimen or a similar regimen.

The above described decision algorithm has been developed and tested on cancer patients following a FOLFOX 4, FOLOFOX 6 or FOLFOX 7 regimen:
FOLFOX 4 (de Gramont A, et al: J Clin Oncol 15: 808-815, 1997; Maindrault-Goebel F, et al. Ann Oncol. 2000 Nov;11 (11):1477-83) : a cycle every 15 days comprising :
5-FU bolus 400 mg/m²
   + elvorine (calcium folinate, 100 mg/m²) at day 1
   + 5-FU 46 hours (initial dose D(1) = 2500 mg/m² or less if the patient has an increased sensitivity to 5-FU, see below) starting on day 1 and stopping on day 2
   + oxaliplatin 85 mg/m² at day 1
FOLFOX 6 (Tournigand C, et al. J Clin Oncol 2004 Jan 15; 22(7):229-37; Maindrault-Goebel F, et al. Ann Oncol. 2000 Nov;11(11):1477-83) : a cycle every 15 days comprising :
   5-FU bolus 400 mg/m²
   + elvorine (calcium folinate, 100 mg/m²) at day 1
   + 5-FU 46 hours (initial dose D(1) = 2500 mg/m² or less if the patient has an increased sensitivity to 5-FU, see below) starting on day 1 and stopping on day 2
   + oxaliplatin 100 mg/m² at day 1
FOLFOX 7 (Maindrault-Goebel F, et al. Eur J Cancer. 2001 May;37(8):1000-5) : a cycle every 15 days comprising :
   5-FU bolus 400 mg/m²
   + elvorine (calcium folinate, 100 mg/m²) at day 1
   + 5-FU 46 hours (initial dose D(1) = 2500 mg/m² or less if the patient has an increased sensitivity to 5-FU, see below) starting on day 1 and stopping on day 2
   + oxaliplatin 130 mg/m² at day 1

As mentioned in the background section, algorithms for optimizing 5-FU dose cannot be transposed from a particular treatment regimen to another really different specific treatment regimen.

Since the above described algorithm has been elaborated and tested on cancer patients treated with (FOLFOX regimens above) it is accurate for these particular regimens and for similar regimens. Indeed, parameters such as the duration of the 5-FU continuous infusion, the presence of folinic acid or of oxaliplatin cannot be significantly changed. However, a small variation in these parameters does not impair the accuracy of the decision algorithm.

Regimens similar to FOLFOX regimens can thus be defined as regimens comprising repeated treatment cycles, two successive cycles being usually separated by about two weeks (cycles are separated by two weeks in normal cases. However, in case of significant toxicity observed after a particular cycle, the next cycle may be delayed of about one or several weeks, thus separating the two cycles of about three weeks or more), each treatment cycle comprising:
- 0-500 mg/m² of 5-fluorouracile (5-FU) administered in a bolus,
- 0-600 mg/m² of folinic acid or a salt thereof,
- a dose D(i) (in mg/m²) of 5-FU administered in a continuous infusion of 43 to 49 hours, and
- 70-130 mg/m² of oxaliplatin.

Each cycle i is preferably identical to the previous cycle (i-1), except for the 5-FU dose D(i) administered in a continuous infusion, which is optimized based on the plasmatic 5-FU concentration measured from said patient blood sample taken during the 5-FU continuous infusion of the previous cycle and on the above described decision algorithm.

As mentioned before, the duration of the 5-FU continuous infusion may not be significantly changed compared to the 46 hours of the FOLFOX protocols. However, a 5-10% variation does not impair the algorithm accuracy, and the duration of the 5-FU continuous infusion may thus be comprised between 43 and 49 hours. In preferred embodiments, the duration of the 5-FU continuous infusion is however comprised between 44 and 48 hours, preferably 45 to 47 hours, and most preferably is about 46 hours. According to the invention, the term "about", when applied to a time period, is intended to mean an increase or decrease of half an hour around the specified value.

The particular treatment regimens on which the above described decision algorithm has been elaborated and tested comprise in each cycle i a 5-FU bolus of 400 mg/m². However, contrary to the presence of folinic acid or of oxaliplatin in treatment cycles, the presence of a 5-FU bolus is not a critical parameter for the accuracy of the decision algorithm.

Indeed, when it is present, said 5-FU bolus is administered before the beginning of the 5-FU continuous infusion. Usually, when a 5-FU bolus is administered, then the 43-49 hours 5-FU infusion is just following the 5-FU bolus. In addition, 5-FU has a very short half-life in blood, and 5-FU plasmatic concentration thus very rapidly decrease after the end of the 5-FU bolus, so that the 5-FU bolus dose does not affect the 5-FU plateau plasmatic concentration during the continuous infusion and has thus no influence on the decision algorithm, provided however that the 5-FU bolus dose does not exceed 500 mg/m². However, in preferred embodiments, each treatment cycle i is such that a dose of 5-FU of about 400 mg/m² is administered in a bolus, as in known FOLFOX regimens.

The term "about", when applied to any therapeutic agent dose (including 5-FU, folinic acid, and oxaliplatin), is intended to mean an increase or decrease of 10% around the specified value.

As mentioned before, folinic acid is necessarily present in each treatment cycle i. Folinic acid, i.e. N-(5-formyl-(6R,S)-5,6,7,8- tetrahydropteroyl)-L-glutamic acid, when obtained by chemical synthesis, is formed by an equimolar mixture of its two (6R) (also called D-folinic acid because this isomer is dextrogyre) and (6S) (also called L-folinic acid because this isomer is levogyre) diastereomeric forms. It is known that only the (6,S) isomer, has the well-known pharmacological activity of the product, while the other one is totally devoid of it. Although folinic acid or a salt thereof may be administered as a racemate mixture of L- and D-folinic acid, any dose of folinic acid or salt thereof is thus expressed as a dose of L-folinic acid. Thus, when a range of 0-600 mg/m² of folinic acid is mentioned, then it means that a dose of 0-600 mg/m² of L-folinic acid is administered to the patient. As a result, if a racemate mixture of L- and D-folinic acid is administered, then the total (L- and D-folinic acid) dose of folinic acid is comprised between 0-1200 mg/m² so that the dose of L-folinic acid be comprised between 0-600 mg/m².

In the particular treatment regimens on which the above described decision algorithm has been elaborated and tested, the dose of folinic acid (i.e. the dose of L-folinic acid) is 100 mg/m². The decision algorithm can thus be relevant for a dose of 0-600 mg/m². In preferred embodiment, the dose of folinic acid administered in each cycle i is comprised between 24-360 mg/m², preferably 45-240 mg/m², more preferably 56-180 mg/m², even more preferably 80-120 mg/m². Most preferably, the dose of folinic acid administered in each cycle i is about 100 mg/m², as in known FOLFOX regimen.

As mentioned before, oxaliplatin is also necessarily present in each treatment cycle i. Since the above described decision algorithm has been elaborated and tested on three distinct FOLFOX regimens (FOLFOX 4, FOLFOX 6, and FOLFOX 7) with distinct amounts of oxaliplatin, the decision algorithm can be generalized to any treatment regimen with the above described parameters and with an oxaliplatin dose administered in each cycle comprised between 70 and 130 mg/m², preferably between 85 and 130 mg/m². The dose of oxaliplatin administered to the patient in each cycle i is about 85, 100 or 130 mg/m², as in known FOLFOX 4, FOLFOX 6 and FOLFOX 7 regimens.

In addition, the decision algorithm has been further validated in patients treated with a FOLFOX 4, FOLFOX 6 or FOLFOX 7 regimen to which is added the administration of a monoclonal antibody (cetuximab or panitumumab) directed to EGFR (epidermal growth factor receptor), or a monoclonal antibody (bevacizumab) directed to VEGF (vascular endothelial growth factor). Thus, in a further embodiment of the method according to the invention described above, the treatment further comprises in each cycle i the administration to the patient of a anticancer monoclonal antibody, preferably a monoclonal antibody directed to EGFR or VEGF, preferably cetuximab, panitumumab or bevacizumab.

In the present disclosure, "D(i)" always refers to the 5-FU dose administered to the patient at cycle i in a continuous infusion of 43 to 49 hours. The determination of the next dose D(n+1) to be administered at cycle (n+1) depends on the previous dose D(n) administered at cycle n, and on the value of the 5-FU plasmatic concentration ([5-FU]) measured from a patient blood sample taken during the 5-FU continuous infusion of previous cycle n. To be representative, the measured 5-FU plasmatic concentration has to be a plateau 5-FU plasmatic concentration.

5-FU has a very short half-life in blood, and 5-FU plasmatic concentration thus very rapidly decrease after the end of the 5-FU continuous infusion. As a result, to be representative, the blood sample taken from the patient in previous cycle n has to be taken before the end of the continuous infusion, and not after.

In addition, 5-FU plasmatic levels normally reach a plateau about 1 hour after the beginning of the 5-FU continuous infusion. For more security, it is sometimes considered that waiting for 1 hour and a half after the beginning of the 5-FU continuous infusion permits to be sure that the plateau has been reached by most patients. As a result, the blood sample may be taken at least 1 hour, preferably at least 1 hour and a half and even more preferably at least 2 hours after the beginning of the continuous infusion and before the end of said continuous infusion.

However, in some patients, the time necessary to reach a 5-FU plasmatic concentration plateau is higher. Thus, the blood sample is taken in previous cycle n in the second half of the 5-FU continuous infusion. Advantageously, the blood sample has thus been taken in cycle n 15 minutes to 22 hours, preferably 30 minutes to 10 hours, more preferably 1 hour to 5 hours, and most preferably 2 to 3 hours before the end of the 5-FU continuous infusion.

Alternatively, since the plateau is generally reached about 1 hour after the beginning of the 5-FU continuous infusion, it may be beneficial for other aspects to take the blood sample for pharmacokinetics analysis in the plateau as soon as possible after the beginning of the 5-FU continuous infusion, i.e. as soon as possible after at least 1 hour, preferably at least 1 hour and a half and even more preferably 2 hours after the beginning of the continuous infusion. Indeed, the continuous infusion is for about 46 hours, and patients usually wish to stay the shortest time possible in the hospital. While the set up of the 5-FU continuous infusion should be done by a qualified person in the hospital, there are currently delivery devices that may then permit to the patient to go home and stay at home during the rest of the continuous infusion. This would then permit to significantly improve patients' quality of life, provided that the blood sample necessary for pharmacokinetics analysis and calculation of the next 5-FU dose by continuous infusion be taken before the patient leaves the hospital.

Since 5-FU plasmatic levels normally reach a plateau about 1 hour after the beginning of the 5-FU continuous infusion, in another preferred example, the blood sample is taken in previous cycle n at least 1 hour after the beginning of the 5-FU continuous infusion and but in the first half of the continuous infusion, i.e. between 1 hour and 23 hours after the beginning of the 5-FU continuous infusion, preferably between 1 hour and a half and 10 hours after the beginning of the 5-FU continuous infusion, preferably between 1 hour and a half and 5 hours after the beginning of the 5-FU continuous infusion, preferably between 1 hour and a half and 3 hours after the beginning of the 5-FU continuous infusion, or between 2 hours and 5 hours after the beginning of the 5-FU continuous infusion, preferably between 2 hours and 4 hours or between 2 hours and 3 hours after the beginning of the 5-FU continuous infusion.

In the regimens of cancer patients, an initial 5-FU dose D(1) has to be administered in a continuous infusion of 43-49 hours in cycle 1. This dose is normally fixed to a standard dose of about 2500 mg/m² (which is the standard dose used in FOLFOX regimens), except in cases in which the patient has been determined to display an increased sensitivity to 5-FU.

By "increased sensitivity to 5-FU" is meant an increase in said subject, compared to a control subject, of the percentage of 5-FU that is metabolized by the anabolic pathway. In a "control subject", only 20% of administered 5-FU is metabolized by the anabolic pathway, while 80% of administered 5-FU is metabolized by DPD in the catabolic pathway. In a patient with an increased sensitivity to 5-FU, the percentage of 5-FU that is metabolized by the anabolic pathway is increased due to a total or partial DPD deficiency and preferably at least 40%, at least 60%, at least 80%, at least 90%, or at least 95% of administered 5-FU is metabolized by the anabolic pathway.

In the case of a patient with an increased sensitivity to 5-FU, the initial dose D(1) is decreased, and the decision algorithm is then applied in the same manner. This way, there is no risk of high grade toxicity, and only benign grade I toxicities should be obtained at worse. The method according to the invention using the decision algorithm then permits to optimize the 5-FU dose D(i) at each cycle in order to reach the maximal tolerable dose.

Thus, the patient has preferably been subjected to the diagnosis of increased 5-FU sensitivity before the beginning of the treatment, and the initial dose D(1) is determined depending on the obtained diagnosis.

In a preferred example of the method according to the invention, the 5-FU dose D(1) administered in a continuous infusion in cycle 1 is at most about 2500 mg/m² and has been determined based on the pre-treatment diagnosis of a possible increased sensitivity of said patient to 5-FU.

EP 1 712 643 application relates to methods for diagnosing an increased sensitivity to 5-FU of a subject.

Briefly, the diagnosis of increased sensitivity of said patient to 5-FU is preferably performed from at least one biological sample of said patient by combining at least two of the following in vitro tests:
a) the analysis of the presence of a significant mutation in DPD gene,
b) the measure of uracil plasmatic concentration, and
c) the measure of the ratio dihydrouracil plasmatic concentrations / uracil plasmatic concentration (UH₂/U ratio).

By a "biological sample" is meant any sample taken from the patient, including a blood sample, an organ sample (a biopsy for instance), a bone marrow sample, etc. For measuring the uracil and dihydrouracil plasmatic concentrations, said biological sample is preferably a blood or plasma sample. For the analysis of the presence of a significant mutation in DPD gene, said sample may be any biological sample from said patient comprising nucleated cells, including a blood sample, an organ sample (for instance cells isolated from a partially metastased lymph node taken from said patient). Preferably, in all cases, said biological sample is a blood or plasma sample.

A "mutation" in DPD gene means any modification of the nucleic sequence of DPD gene, including substitutions (transversions as well as transitions), deletions and insertions.

A "significant mutation" in DPD gene is defined as a mutation that generates a decrease of DPD enzymatic activity. Preferably, a significant mutation in DPD gene results in a decrease of DPD enzymatic activity of at least 50%, at least 60%, at least 70%, at least 80%, or at least 90% of DPD enzymatic activity. Such mutations are known to skilled artisan. Notably, the mutations in DPD gene of following Table 1 are considered as significant mutations of the DPD gene

**Table 1. Known significant mutations in DPD gene**

| Mutation | Position in DPD gene | Consequence at DPD gene level | Consequence at DPD protein level |
|---|---|---|---|
| R21 Stop (=C61T) | exon 2 | Substitution of cytosine by thymine in position 61 | Early stop codon => no DPD activity |
| Del TCAT295 | exon 4 | Deletion of 4 bases in position 295 | Early stop codon => no DPD activity |
| L155Stop (=T464A) | exon 5 | Substitution of thymine by adenine in position 464 | Early stop codon => no DPD activity |
| Del T812 | exon 8 | Deletion of thymine in position 812 | Early stop codon => no DPD activity |
| Del TG1039 | exon 10 | Deletion of 4 bases in position 1039 | Early stop codon => no DPD activity |
| E386Stop (=G1156T ) | exon 11, codon 386 | Substitution of guanine by thymine in position 1156 | Early stop codon => no DPD activity |
| I560S (=T1679G ) | exon 13, | Substitution of thymine by guanine in position 1679 | conformational change=> partial or complete loss of DPD activity |
| Del C1897 | exon 14 | Deletion of cytosine in position 1897 | stop codon at the DPD substrate binding site => complete loss in DPD activity in a patient |
| IVS14+1G >A | intron 14 | Substitution of guanine by adenine at intron beginning | complete deletion of exon 14 during pre-messenger RNA transcription (loss of 165 bp) => complete loss of DPD activity |
| D949V (=A2846T ) | exon 22 | Substitution of adenine by thymine in position 2846 | Direct interference with cofactor binding or electron transport, altered [4Fe-4S] function |

Significant mutations such as those described in Table 1 may be detected from a blood sample using any method known by those skilled in the art. For instance, hybridization probes and assays, microarrays or sequencing may be used.

The uracil and dihydrouracil plasmatic concentrations may be measured from a blood or plasma sample using any technology known to those skilled in the art. Notably, these concentrations may be measured from a blood or plasma sample using HPLC with UV-detection, using a HPLC column with a stationary phase composed of totally porous spherical carbon particles such as Hypercarb™ columns sold by Thermo Electron (Courtaboeuf, France).

Still more preferably, in a method according to the invention including the diagnosis of an increased sensitivity of said patient to 5-FU from at least one biological sample of said patient by combining at least two of in vitro tests, all three in vitro test have been performed and the initial dose D(1) has been determined using the following decision algorithm:
(a) If
   - no significant mutation in DPD gene has been detected and uracil plasmatic concentration is less than 15 µg/L, or
   - no significant mutation in DPD gene has been detected and uracil plasmatic concentration is at least 15 µg/L but the UH₂/U ratio is at least 6,
      then a standard dose D(1) of 2500 mg/m² is administered to the patient in cycle 1.
(b) In all other cases,
   - if 6 ≤ UH₂/U ratio, then D(1) is 1750 mg/m²
   - if 3 ≤ UH₂/U ratio < 6, then D(1) is 1250 mg/m²
   - if 1 ≤ UH₂/U ratio < 3, then D(1) is 750 mg/m²
   - if UH₂/U ratio < 1, then the patient is preferably not treated with 5-FU.

Using such a protocol for detecting patients with increased sensitivity o 5-FU before any 5-FU administration, the initial 5-FU dose D(1) administered in cycle 1 is adapted and no severe toxicity is normally observed. More precisely, using this protocol of early increased sensitivity o 5-FU detection and dose adaptation, no toxicity or only grade 1 toxicities are usually observed after the first treatment cycle.

The above described methods in which the next 5-FU dose administered in a 43-49 hours infusion in the next cycle can then usually by applied without the observation of toxicities of at least grade 2. Since DPD deficiency is really the major factor involved in 5-FU toxicity, the early detection of increased 5-FU sensitivity and the adaptation of the first cycle dose D(1) of 5-FU administered in a 43-49 hours infusion permits to prevent the occurrence of at least grade 2 toxicities in almost all cases. The above described methods according to the invention can thus be applied without any modification in almost all cases.

However, if in very rare cases, toxicities of at least grade 2 are observed, then the following protocol described in Table 2 below may be used depending on the type of observed toxicity:

| | **Toxicity type** | **Initial dose(mg/m²/cycle)** | |
|---|---|---|---|
| | | **5-FU Bolus** | **5-FU 43-49 h continuous infusion** |
| | **(CTCAE V3.0 Grade)** | 400 mg/m² day 1 | 2500 mg/m² or less if a increased sensitivity to 5-FU has been detected |
| | | 5-FU dose adaptation at cycle 2 (mg/m²/cycle) | |
| | Anemia (all grades) | No modification | No modification |
| Nausea and/or vomiting Grade 4 in spite of pre-medication | | Adapted anti-emetic therapy | |
| | | Treatment stopped if not tolerable | |
| | Neutropenia ou Thrombopenia Grade 3 for 4 | 300 then 200 * | 20% decrease** |
| | Febril Neutropenia defined as fever grade 2 (oral measure ≥ 38°C or 3 elevations ≥ 38°1C in 24 hours), associated to a grade 4 neutropenia. | | |
| | Diarrhea Grade 3 or 4 | | |
| | Stomatitis Grade 3 or 4 | | |
| | Cardiac toxicity ≥ Grade 2 | Treatment stopped | |
| | Cutaneous toxicity Grade 3 or 4 | 300 then 200 * | 20% decrease 20% ** |
| | Allergy Grade 3 or 4 | Treatment stopped | |
| | Neurocérébelleuse | Treatment stopped | |
| | Alopecia (all Grades) | No modification | No modification |
| Local tolerance (all Grades) | | No modification | No modification |
| Other toxicity clearly linked to a chemotherapeutic drug | | | |
| | - Grade I and 2 | No modification | No modification |
| | - Grade 3 | 300 then 200 * | 20% decrease ** |
| | - Grade 4 | Treatment stopped | Treatment stopped |

| | | | |
|---|---|---|---|
| *5-FU : In case of hematological toxicity recurrence after two dose reductions, the administration of a 5-FU bolus is stopped. Folinic acid : folinic acid doses are usually not modified. ** : compared to the former 5-FU dose (in mg) administered in the preceding treatment cycle | | | |

The methods according to the invention are intended for patients suffering from diseases that may be treated using a FOLFOX regimen or a similar regimen. Such diseases notably include colorectal cancer, stomach cancer, hepatic ducts cancer, pancreas cancer, oesophagus cancer, or breast cancer.

### DESCRIPTION OF THE DRAWINGS

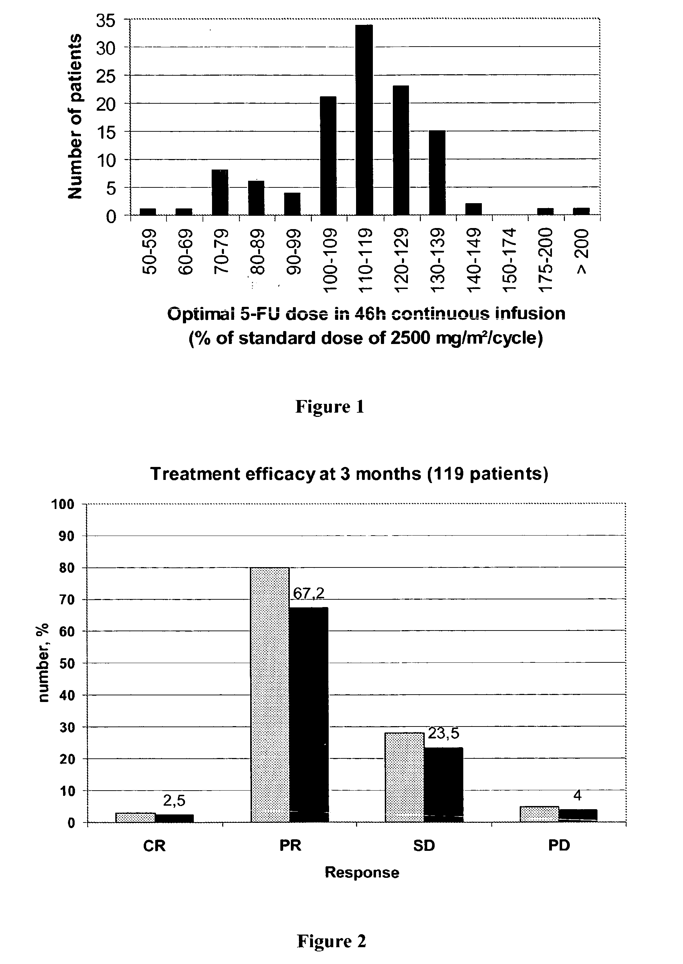

**Figure 1****.** Distribution of optimal 5-FU doses in 46 hours continuous infusion. For each range of optimal dose, the optimal dose is expressed as a percentage of the standard dose of 2500 mg/m²/cycle and the number of patients for which the dose was found optimal is indicated.
**Figure 2****.** Treatment efficiency at 3 months in 119 patients. The number (grey bars) and percentage (black bars) of 119 patients treated during 3 months using the 5-FU adaptation method according to the invention displaying at 3 months a complete response to the treatment (CR), a partial response to the treatment (PR), a stable disease (SD) or a progressive disease (PD) are represented. Percentages of patients are indicated over their respective black bars.

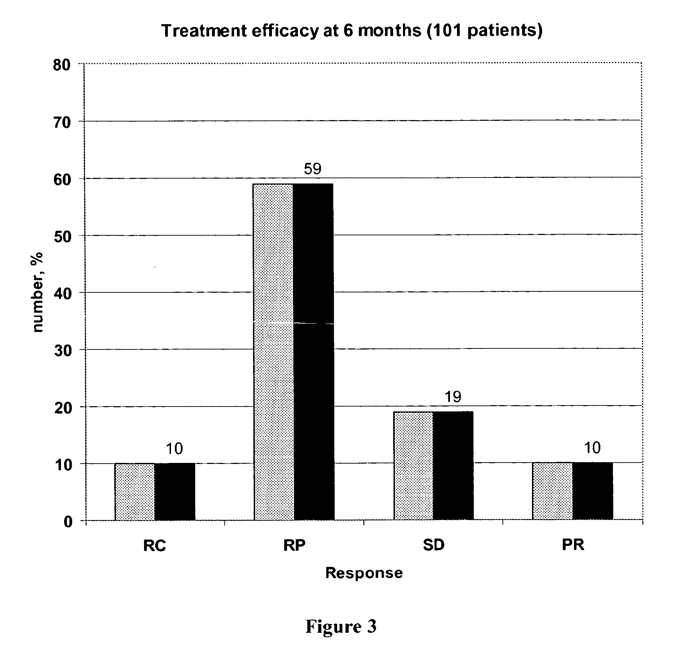

**Figure 3****.** Treatment efficiency at 6 months in 101 patients. The number (grey bars) and percentage (black bars) of 101 patients treated during 6 months using the 5-FU adaptation method according to the invention displaying at 6 months a complete response to the treatment (CR), a partial response to the treatment (PR), a stable disease (SD) or a progressive disease (PD) are represented. Percentages of patients are indicated over their respective black bars.

### EXAMPLES

The claimed method was used in a study comprising 119 patients treated by a FOLFOX regimen in order to determine the capacity of the method to increase efficiency of the treatment and to decrease treatment toxicity.

### Patients and methods

### Patients

The features of the 119 tested patients are described in following Table 3:

| **Characteristics** | **Number of patients** |
|---|---|
| **Age (mean ± se)** | **63± 10** |

| **Primary tumor** | |
|---|---|
| **Colon** | **79** |
| **Rectum** | **40** |

| **Metastasis Number of sites :** | |
|---|---|
| **1** | **75** |
| **2** | **33** |
| **3** | **5** |
| **>3** | **5** |

| **Metastasis sites** | |
|---|---|
| **Liver** | **57** |
| **Lung** | **5** |
| **Both** | **41** |
| **>2** | **16** |

| **Metastasis** | |
|---|---|
| **Synchrone** | **109** |
| **asynchrone** | **10** |

| **Performance status** | |
|---|---|
| **0** | **68** |
| **1** | **37** |
| **2** | **14** |

### Treatment administered

Patients were first diagnosed for the presence of a possible increased sensitivity to 5-FU according to the method described above in the general description.

Patients were then treated following one of the three FOLFOX 4, FOLFOX 6 or FOLFOX 7 regimen described above in the general description, except that the initial 5-FU dose administered in a 46 hours continuous infusion was adapted if necessary depending on the diagnosis of increased 5-FU sensitivity.

At each cycle, the next 5-FU dose for the 46 hours continuous infusion was calculated according to the method of the present invention.

### Results

### Initial 5-FU dose for the 46 hours continuous infusion

Based on the previous detection of a possible increased sensitivity to 5-FU, the initial 5-FU dose for the 46 hours continuous infusion of the first cycle was adapted as follows:

**Table 4. Adaptation of the initial 5-FU dose for the 46 hours continuous infusion**

| **D (cycle 1) (% of standard)** | **Number of patients** | **% of patients** |
|---|---|---|
| ≥ 100 | 96 | 80,7 |
| 50<D<100 | 16 | 13,4 |
| ≤ 50 | 6 | 5,1 |

### Optimal 5-FU dose for the 46 hours continuous infusion

Using the adaptation method according to the invention permitting to calculate at each cycle the next 5-FU dose for the 46 hours continuous infusion, the dose of each patient was stabilized to an optimal dose.

The range of obtained optimal doses, expressed as the percentage of the standard dose 2500 mg/m²/cycle, is represented in Figure 1, and in following Table 5:

**Table 5. Optimal 5-FU dose for the 46 hours continuous infusion**

| **Doptimal (% of standard)** | **Number of patients** | **% of patients** |
|---|---|---|
| < 90 | 16 | 13.7 |
| 90 ≤D ≤110 | 46 | 39.3 |
| > 110 | 55 | 47.0 |
| > 120 | 27 | 23.1 |

Results clearly show that the optimal dose has been changed from the standard dose (± 10%) in most patients (60.7%). More precisely, the optimal dose is:
- decreased by more than 10% (optimal dose < 2250, 13.7% of patients) or increased by more than 10% (optimal dose > 2750, 47.0% of patients) compared to the standard dose in 60.7% of patients. In addition, the optimal dose was increase by more than 20% (optimal dose > 3000) compared to the standard dose in 23.1% of patients, which represents a significant proportion of patients.
- maintained to the standard dose +/- 10% in only 39.3% of patients.

These results highlight the inadequacy of standard doses and thus the importance of the method according to the invention.

### Objective response

The treatment efficiency at 3 and 6 months is displayed in Figures 2 and 3 respectively.

While with standard FOLFOX regimens (without 5-FU dose adaptation) an objective response (complete response (CR) or partial response (PR)) of 40-45% is usually observed at 3 months, patients treated with the adaptive method according to the invention had a significantly increased objective response of 69.7% (see Figure 2).

At 6 months, the objective response of patients treated with the adaptive method according to the invention was still of 69% (see Figure 3).

Data concerning patients overall survival and progression-free survival are summarized in following Table 6:

**Table 6. Patients survival statistics**

| **Progression free survival** | |
|---|---|
| Mean (mths) | 19 |
| Median (mths) | 16 |
| At 1 year | 56% |
| At 2 years | 37% |

| **Overall survival** | |
|---|---|
| Mean (mths) | 32 |
| Median (mths) | 28 |
| At 1 year | 87% |
| At 2 years | 57.5% |

As mentioned above, optimal doses had to be adapted in most patients. In particular, about a quarter of all patients received an optimal 5-FU dose in the 46 hours continuous infusion of more than 3000 mg/m²/cycle. Obviously, if the 5-FU dose in the 46 hours continuous infusion has not been increased in these patients, they would have received a too low suboptimal dose of 5-FU and would not have responded to the treatment.

The obtained results thus clearly show that the 5-FU adaptation method according to the invention permits to significantly increase the efficiency of the treatment.

### Toxicity

In addition to the increase treatment efficiency, the 5-FU adaptation method according to the invention also permitted to significantly decrease the observed toxicities induced by the treatment.

At 3 months, toxicities were as described in following table 7:

**Table 7. Toxicity at 3 months**

| **At 3 months** | **Number of patients** | **Percentage of patients** |
|---|---|---|
| toxicity grade 0 (no toxicity) | 115 | 96.6 |
| toxicity grade 1 | 2 | 1.68 |
| toxicity grade 2 | 1 | 0.84 |
| toxicity grade 3 | 1 | 0.84 |
| toxicity grades 0 or 1 | 117 | 98.3 |
| toxicity grades 2 or 3 | 2 | 1,7 |

During the whole treatment, toxicities were as described in following table 8:

**Table 8. Toxicity during the whole treatment**

| **During the whole treatment** | **Number of patients** | **Percentage of patients** |
|---|---|---|
| toxicity grade 0 (no toxicity) | 108 | 92,3 |
| toxicity grade 1 | 5 | 4,3 |
| toxicity grade 2 | 2 | 1,7 |
| toxicity grade 3 | 2 | 1,7 |
| toxicity grades 0 or 1 | 113 | 96,6 |
| toxicity grades 2 or 3 | 4 | 3,4 |

Thus, while toxicities due to 5-FU are usually observed in 20-25% of patients using a standard FOLFOX protocol, only 7.7% and 3.4% of patients treated with the 5-FU adaptation method according to the invention displayed a toxicity (all grades or grades 2 and 3) respectively.

### Conclusion

In view of the above results, it is thus clear that the 5-FU adaptation method according to the invention permits to significantly improve patients treatment by simultaneously increasing treatment efficiency (and thus the percentage of objective response) and decreasing toxicities due to 5-FU administration (thus improving patients quality of life).

### BIBLIOGRAPHY

de Gramont A, Bosset JF, Milan C, et al: Randomized trial comparing monthly low-dose leucovorin and fluorouracil bolus with bimonthly high-dose leucovorin and fluorouracil bolus plus continuous infusion for advanced colorectal cancer: a french intergroup study. J Clin Oncol 15: 808-815, 1997

EP 1 712 643

Etienne M C, Lagrange J L, Dassonville O, et al: Population study of dihydropyrimidine dehydrogenase in cancer patients. J Clin Oncol 12: 2248-2253, 1994

Gamelin E., Boisdron-Celle M., Guérin-Meyer V., Delva R., Lortholary A., Genevieve F., Larra F., Ifrah N., Robert J. Correlation between uracil and dihydrouracil plasma ratio, and 5-fluorouracil pharmacokinetic parameters and tolerance in patients with advanced colorectal cancer. A potential interest for predicting 5-FU toxicity and for determining optimal 5-FU dosage. J Clin Oncol, 1999, 17, 1105-1110

Gamelin E., Boisdron-Celle M., Delva R., Regimbeau C., Cailleux P.E., Alleaume C., Maillet M.L., Goudier M.J., Sire M., et al. Long-term weekly treatment of colorectal metastatic cancer with fluorouracil and leucovorin : results of a multicentric prospective trial of fluorouracil dosage optimization by pharmacokinetic monitoring in 152 patients. J. Clin. Oncol., 1998, 16 (4), 1470-1478

Gamelin E., Boisdron-Celle M. Dose monitoring of 5-fluorouracil in patients with colorectal or head and neck cancer. Status of the art. Crit Rev Oncol Hematol, 1999, 30, 71-79

Gamelin E, Delva R, Jacob J, Merrouche Y, Raoul JL, Pezet D, Dorval E, Piot G, Morel A, Boisdron-Celle M. Individual fluorouracil dose adjustment based on pharmacokinetic follow-up compared with conventional dosage: results of a multicenter randomized trial of patients with metastatic colorectal cancer. J Clin Oncol. 2008 May 1;26(13):2099-105.

Graham J, Mushin M, Kirkpatrick P. Oxaliplatin. Nat Rev Drug Discov, 2004, 3: 11-12

Maindrault-Goebel F, de Gramont A, Louvet C, André T, Carola E, Gilles V, Lotz JP, Tournigand C, Mabro M, Molitor JL, Artru P, Izrael V, Krulik M. dose intensity in bimonthly leucovorin and 48-hour 5-fluorouracil continuous infusion regimens (FOLFOX) in pretreated metastatic colorectal cancer. Oncology Multidisciplinary Research Group (GERCOR).Ann Oncol. 2000 Nov; 11(11):1477-83.

Maindrault-Goebel F, de Gramont A, Louvet C, André T, Carola E, Mabro M, Artru P, Gilles V, Lotz JP, Izrael V, Krulik M; Oncology Multidisciplinary Research Group (GERCOR). High-dose intensity oxaliplatin added to the simplified bimonthly leucovorin and 5-fluorouracil regimen as second-line therapy for metastatic colorectal cancer (FOLFOX 7). Eur J Cancer. 2001 May;37(8):1000-5.

Tournigand C, Andre T, Achille E et al. FOLFIRI followed by FOLFOX6 or the reverse sequence in advanced colorectal cancer : a randomized GERCOR study. J Clin Oncol 2004 Jan 15; 22(7):229-37

Ychou M, Duffour J, Kramar A, et al. Individual 5-FU adaptation in metastatic colorectalo cancer: results of a phase II study using a bimonthly pharmacokinetically intensified LV5FU2 regimen. Cancer Chemother Pharmacol, 2003, 52: 282-90.

## Claims

1. A method for determining from a blood sample of a patient suffering from cancer the dose D(n+1) of 5-fluorouracile (5-FU) for the next cycle of treatment (n+1), wherein
• each treatment cycle i comprises:
- 0-500 mg/m² of 5-fluorouracile (5-FU) administered in a bolus,
- 0-600 mg/m² (plus ou moins 20%) of folinic acid or a salt thereof,
- a dose D(i) (in mg/m²) of 5-flu administered in a continuous infusion of 43 to 49 hours, and
- 70-130 mg/m² of oxaliplatin; and
• said blood sample has been taken from said patient in previous treatment cycle n at least 1 hour after the beginning of the 5-FU perfusion and before the end of said perfusion,
• said method comprising:
• dosing in vitro the 5-FU plasmatic concentration ([5-FU]) in the blood sample
• calculating D(n+1) depending on D(n) using the following decision scheme:
- if [5-FU] < 100 µg/L, then D(n+1) = D(n) × 1.40,
- if 100 ≤ [5-FU] < 200 µg/L, then D(n+1) = D(n) × 1.30,
- if 200 ≤ [5-FU] < 300 µg/L, then D(n+1) = D(n) × 1.20,
- if 300 × [5-FU] < 400 µg/L, then D(n+1) = D(n) × 1.10,
- if 400 ≤ [5-FU] < 550 µg/L, then D(n+1) = D(n) × 1.05,
- if 550 ≤ [5-FU] ≤ 600 µg/L, then D(n+1) = D(n),
- if 600 < [5-FU] < 700 µg/L, then D(n+1) = D(n) × 0.95,
- if 700 ≤ [5-FU] < 800 µg/L, then D(n+1) = D(n) × 0.90,
- if 800 ≤ [5-FU] < 900 µg/L, then D(n+1) = D(n) × 0.85,
- if [5-FU] ≥ 900, then D(n+1) = D(n) × 0.80,

2. The method according to claim 1. wherein the duration of the continuous infusion of 5-FU in each cycle i is 46 hours.

3. The method according to claim 1 or 2, wherein the dose of 5-FU administered in a bolus in each cycle i is 400 mg/m².

4. The method according to anyone of claims 1-3, wherein the dose of folinic acid or salt thereof administered to the patient in each cycle i is 100 mg/m².

5. The method according to anyone of claims 1-4, wherein the dose of oxaliplatin administered to the patient in each cycle i is 85, 100 or 130 mg/m^{2:}

6. The method according to anyone of claims 1-5, wherein the treatment further comprises the administration to the patient in each cycle i of an anticancer monoclonal antibody.

7. The method according to claim 6, wherein said anticancer monoclonal antibody is cetuximab, panitumumab or bevacizumab.

8. The method according to anyone of claims 1-7, wherein the blood sample has been taken in cycle n 15 minutes to 22 hours before the end of the 5-FU continuous infusion.

9. The method according to claim 8, wherein the blood sample has been taken in cycle n 2 to 3 hours before the end of the 5-FU continuous infusion.

10. The method according to anyone of claims 1-7, wherein the blood sample has been taken in cycle n between 1 hour and 5 hours after the beginning of the 5-FU continuous infusion.

11. The method according to anyone of claims 1-10, wherein the 5-FU dose D(1) administered in a continuous infusion in cycle I is at most 2500 mg/m² and has been determined based on the pre-treatment diagnosis of a possible increased sensitivity of said patient to 5-FU.

12. The method according to claim 11, wherein the diagnosis of a possible hypersensitivity of said patient to 5-FU is performed from at least one biological sample of said patient by combining at least two of the following in vitro tests:
a) the analysis of the presence of a significant mutation in DPD gene,
b) the measure of uracil plasmatic concentration, and
c) the measure of the ratio dihydrouracil plasmatic concentrations /uracil plasmatic concentration (UH₂/U ratio).

13. The method according to claim 12, wherein the three in vitro test have been performed and the initial dose D(1) has been determined using the following decision algorithm:
a) If
- no significant mutation in DPD gene has been detected and uracil plasmatic concentration is less than 15 µg/L, or
- no significant mutation in DPD gene has been detected and uracil plasmatic concentration is at least 15 µg/L but the UH₂/U ratio is at least 6,
then a standard dose D(1) of 2500 mg/m² is administered to the patient in cycle 1.
b) In all other cases,
- if 6 ≤ UH₂/U ratio, then D(1) is 1750 mg/m²
- if 3 ≤ UH₂/U ratio < 6, then D(1) is 1250 mg/m²
- if 1 ≤ UH₂/U ratio < 3, then D(1) is 750 mg/m²
- if UH₂/U ratio < 1, then the patient is preferably not treated with 5-FU.

14. The method according to anyone of claims 1-13, wherein said patient is suffering from colorectal cancer, stomach cancer, hepatic ducts cancer, pancreas cancer, oesophagus cancer, or breast cancer.

## Patentansprüche

1. Verfahren zum Ermitteln der Dosis D(n+1) 5-Fluoruracil (5-FU) für den nächsten Behandlungszyklus (n+1) aus einer Blutprobe eines Patienten, der an Krebs erkrankt ist, bei dem
- jeder Behandlungszyklus i Folgendes umfasst:
- 0-500 mg/m² 5-Fluoruracil (5-FU), verabreicht als Bolus,
- 0-600 mg/m² (plus oder minus 20%) Folsäure oder ein Salz davon,
- eine Dosis D(1) (in mg/m²) 5-FU, verabreicht als Dauerinfusion von 43 bis 49 Stunden, und
- 70-130 mg/m² Oxaliplatin; und
- die Blutprobe dem Patienten in dem vorherigen Behandlungszyklus n mindestens 1 Stunde nach Beginn der 5-FU-Infusion und vor dem Ende der Infusion entnommen wurde,
- wobei das Verfahren Folgendes umfasst:
- in-vitro-Dosieren der Plasmakonzentration von 5-FU ([5-FU]) in der Blutprobe
- Berechnen von D(n+1) in Abhängigkeit von D(n) unter Verwendung des folgenden Entscheidungsschemas:
- wenn [5-FU] < 100 µg/l, dann D(n+1) = D(n) x 1,40,
- wenn 100 ≤ [5-FU] < 200 µg/l, dann D(n+1) = D(n) x 1,30,
- wenn 200 ≤ [5-FU] < 300 µg/l, dann D(n+1) = D(n) x 1,20,
- wenn 300 ≤ [5-FU] < 400 µg/l, dann D(n+1) = D(n) x 1,10,
- wenn 400 ≤ [5-FU] < 550 µg/l, dann D(n+1) = D(n) x 1, 05,
- wenn 550 ≤ [5-FU] ≤ 600 µg/l, dann D(n+1) = D(n),
- wenn 600 < [5-FU] < 700 µg/l, dann D(n+1) = D(n) x 0,95,
- wenn 700 ≤ [5-FU] < 800 µg/l, dann D(n+1) = D(n) x 0,90,
- wenn 800 ≤ [5-FU] < 900 µg/l, dann D(n+1) = D(n) x 0,85,
- wenn [5-FU] ≥ 900, dann D(n+1) = D(n) x 0,80.

2. Verfahren nach Anspruch 1, bei dem die Dauer der Dauerinfusion 5-FU in jedem Zyklus i 46 Stunden beträgt.

3. Verfahren nach Anspruch 1 oder 2, bei dem die Dosis 5-FU, die in jedem Zyklus i als Bolus verabreicht wird, 400 mg/m² beträgt.

4. Verfahren nach einem der Ansprüche 1 bis 3, bei dem die Dosis Folsäure oder eines Salzes davon, die dem Patienten in jedem Zyklus i verabreicht wird, 100 mg/m² beträgt.

5. Verfahren nach einem der Ansprüche 1 bis 4, bei dem die Dosis Oxaliplatin, die dem Patienten in jedem Zyklus i verabreicht wird, 85, 100 oder 130 mg/m² beträgt.

6. Verfahren nach einem der Ansprüche 1 bis 5, bei dem die Behandlung ferner das Verabreichen eines monoklonalen Antikörpers zur Krebsbekämpfung an den Patienten in jedem Zyklus i umfasst.

7. Verfahren nach Anspruch 6, bei dem der monoklonale Antikörper zur Krebsbekämpfung Cetuximab, Panitumumab oder Bevacizumab ist.

8. Verfahren nach einem der Ansprüche 1 bis 7, bei dem die Blutprobe in Zyklus n 15 Minuten bis 22 Stunden vor Ende der 5-FU-Dauerinfusion entnommen wurde.

9. Verfahren nach Anspruch 8, bei dem die Blutprobe in Zyklus n 2 bis 3 Stunden vor Ende der 5-FU-Dauerinfusion entnommen wurde.

10. Verfahren nach einem der Ansprüche 1 bis 7, bei dem die Blutprobe in Zyklus n zwischen 1 Stunde und 5 Stunden nach Beginn der 5-FU-Dauerinfusion entnommen wurde.

11. Verfahren nach einem der Ansprüche 1 bis 10, bei dem die 5-FU-Dosis D(I), die als Dauerinfusion in Zyklus 1 verabreicht wird, höchstens 2500 mg/m² beträgt und auf der Grundlage einer vor der Behandlung diagnostizierten möglichen erhöhten Empfindlichkeit des Patienten gegenüber 5-FU ermittelt wurde.

12. Verfahren nach Anspruch 11, bei dem die Diagnose einer möglichen Überempfindlichkeit des Patienten gegenüber 5-FU anhand mindestens einer biologischen Probe des Patienten durch Kombination von mindestens zwei der folgenden in-vitro-Tests erfolgt:
a) die Untersuchung des Vorhandenseins einer erheblichen Mutation im DPD-Gen,
b) die Messung der Plasmakonzentration von Uracil, und
c) die Messung des Verhältnisses der Plasmakonzentration Dihydrouracil / Plasmakonzentration Uracil (UH₂/U-Verhältnis).

13. Verfahren nach Anspruch 12, bei dem die drei in-vitro-Tests durchgeführt wurden und die Anfangsdosis D(I) unter Verwendung des folgenden Entscheidungsalgorithmus ermittelt wurde:
a) wenn
- keine erhebliche Mutation im DPD-Gen nachgewiesen wurde und die Plasmakonzentration von Uracil weniger als 15 µg/l beträgt, oder
- keine erhebliche Mutation im DPD-Gen nachgewiesen wurde und die Plasmakonzentration von Uracil mindestens 15 µg/l beträgt, das UH₂/U-Verhältnis jedoch mindestens 6 beträgt,
wird dem Patienten in Zyklus 1 eine Standarddosis D(I) von 2500 mg/m² verabreicht.
b) In allen anderen Fällen,
- wenn 6 ≤ UH₂/U-Verhältnis, dann beträgt D(I) 1750 mg/m²
- wenn 3 ≤ UH₂/U-Verhältnis < 6, dann beträgt D(I) 1250 mg/m²
- wenn 1 ≤ UH₂/U-Verhältnis < 3, dann beträgt D(I) 750 mg/m²
- wenn UH₂/U-Verhältnis < 1, wird der Patient vorzugsweise nicht mit 5-FU behandelt.

14. Verfahren nach einem der Ansprüche 1 bis 13, bei dem der Patient an einem kolorektalen Karzinom, Magenkrebs, einem Karzinom der Gallengänge, Bauchspeicheldrüsenkrebs, einem Speiseröhrenkarzinom oder Brustkrebs erkrankt ist.

## Revendications

1. Méthode permettant de déterminer à partir d'un échantillon de sang d'un patient souffrant d'un cancer, la dose D(n+1) de 5-fluorouracile (5-FU) pour le cycle suivant de traitement (n+1), dans laquelle
• chaque cycle de traitement i comprend :
- 0 à 500 mg/m² de 5-fluorouracile (5-FU) administrés dans un bolus,
- 0 à 600 mg/m² (plus ou moins 20 %) d'acide folinique ou d'un sel de celui-ci,
- une dose D(i) (en mg/m²) de 5-FU administrée dans une perfusion en continu de 43 à 49 heures, et
- 70 à 130 mg/m² d'oxaliplatine ; et
• ledit échantillon de sang a été prélevé dudit patient dans le cycle de traitement précédent n au moins 1 heure après le début de la perfusion de 5-FU et avant la fin de ladite perfusion,
• ladite méthode comprenant :
• le dosage *in vitro* de la concentration plasmatique de 5-FU ([5-FU]) dans l'échantillon de sang
• le calcul de D(n+1) selon D(n) en utilisant le schéma décisionnel suivant :
- si [5-FU]) < 100 µg/l, alors D(n+1) = D(n) x 1,40,
- si 100 ≤ [5-FU]) < 200 µg/l, alors D(n+1) = D(n) x 1,30,
- si 200 ≤ [5-FU]) < 300 µg/l, alors D(n+1) = D(n) x 1,20,
- si 300 ≤ [5-FU]) < 400 µg/l, alors D(n+1) = D(n) x 1,10,
- si 400 ≤ [5-FU]) < 550 µg/l, alors D(n+1) = D(n) x 1,05,
- si 550 ≤ [5-FU]) < 600 µg/l, alors D(n+1) = D(n),
- si 600 ≤ [5-FU]) < 700 µg/l, alors D(n+1) = D(n) x 0,95,
- si 700 ≤ [5-FU]) < 800 µg/l, alors D(n+1) = D(n) x 0,90,
- si 800 ≤ [5-FU]) < 900 µg/l, alors D(n+1) = D(n) x 0,85,
- si [5-FU]) ≥ 900 µg/l, alors D(n+1) = D(n) x 0,80.

2. Méthode selon la revendication 1, dans laquelle la durée de la perfusion en continu de 5-FU dans chaque cycle i est de 46 heures.

3. Méthode selon la revendication 1 ou 2, dans laquelle la dose de 5-FU administrée dans un bolus dans chaque cycle i est de 400 mg/m².

4. Méthode selon l'une quelconque des revendications 1 à 3, dans laquelle la dose d'acide folinique ou d'un sel de celui-ci administrée au patient dans chaque cycle i est de 100 mg/m².

5. Méthode selon l'une quelconque des revendications 1 à 4, dans laquelle la dose d'oxaliplatine administrée au patient dans chaque cycle i est de 85, 100 ou 130 mg/m².

6. Méthode selon l'une quelconque des revendications 1 à 5, dans laquelle le traitement comprend en outre l'administration au patient dans chaque cycle i d'un anticorps monoclonal anticancéreux.

7. Méthode selon la revendication 6, dans laquelle ledit anticorps monoclonal anticancéreux est le cétuximab, le panitumumab ou le bévacizumab.

8. Méthode selon l'une quelconque des revendications 1 à 7, dans laquelle l'échantillon de sang a été prélevé dans le cycle n 15 minutes à 22 heures avant la fin de la perfusion en continu de 5-FU.

9. Méthode selon la revendication 8, dans laquelle l'échantillon de sang a été prélevé dans le cycle n 2 à 3 heures avant la fin de la perfusion en continu de 5-FU.

10. Méthode selon l'une quelconque des revendications 1 à 7, dans laquelle l'échantillon de sang a été prélevé dans le cycle n entre 1 heure et 5 heures après le début de la perfusion en continu de 5-FU.

11. Méthode selon l'une quelconque des revendications 1 à 10, dans laquelle la dose de 5-FU D(1) administrée dans une perfusion en continu dans le cycle 1 est au plus de 2500 mg/m² et a été déterminée en se basant sur le diagnostic de prétraitement d'une possible sensibilité accrue dudit patient au 5-FU.

12. Méthode selon la revendication 11, dans laquelle le diagnostic d'une hypersensibilité possible dudit patient au 5-FU est réalisé à partir d'au moins un échantillon biologique dudit patient en combinant au moins deux des tests *in vitro* suivants :
a) l'analyse de la présence d'une mutation significative dans un gène DPD,
b) la mesure de la concentration plasmatique en uracile, et
c) la mesure du rapport de la concentration plasmatique de dihydrouracile/la concentration plasmatique en uracile (rapport UH₂/U).

13. Méthode selon la revendication 12, dans laquelle les trois tests *in vitro* ont été réalisés et la dose initiale D(1) a été déterminée en utilisant l'algorithme décisionnel suivant :
a) si
- aucune mutation significative dans le gène DPD n'a été détectée et la concentration plasmatique d'uracile est inférieure à 15 µg/l, ou
- aucune mutation significative dans le gène DPD n'a été détectée et la concentration plasmatique d'uracile est inférieure à 15 µg/l mais le rapport UH₂/U est d'au moins 6,
alors une dose standard D(1) de 2500 mg/m² est administrée au patient dans le cycle 1, b) dans tous les autres cas,
- si 6 ≤ rapport UH₂/U, alors D (1) est de 1750 mg/m²
- si 3 ≤ rapport UH₂/U < 6, alors D (1) est de 1250 mg/m²
- si 1 ≤ rapport UH₂/U < 3, alors D (1) est de 750 mg/m²
- si rapport UH₂/U < 1, alors le patient est de préférence pas traité avec du 5-FU.

14. Méthode selon l'une quelconque des revendications 1 à 13, dans laquelle ledit patient souffre d'un cancer colorectal, d'un cancer de l'estomac, d'un cancer des canaux hépatiques, d'un cancer du pancréas, d'un cancer de l'oesophage ou d'un cancer du sein.
